# EUROPÄISCHE PATENTSCHRIFT

(11) **EP 0 589 325 B1**
(45) Veröffentlichungstag und Bekanntmachung des Hinweises auf die Patenterteilung: **21.05.1997**
(21) Anmeldenummer: 93114701.1
(22) Anmeldetag: 13.09.1993
(51) Int. Cl.: A61F 2/38

(54) **Kniegelenkendoprothese zum Ersatz der Schienbein-Gelenkflächen**
Knee joint endoprosthesis for replacement of the tibial articular surfaces
Endoprothèse de l'articulation du genou pour le remplacement des surfaces articulaires tibiales

(30) Priorität: 24.09.1992 DE 9212879 U
(43) Veröffentlichungstag der Anmeldung: 30.03.1994
(73) Patentinhaber: Waldemar Link (GmbH & Co.), D-22339 Hamburg (DE)
(72) Erfinder: Keller, Arnold, D-23863 Kayhude (DE)
(74) Vertreter: Glawe, Delfs, Moll & Partner

(56) Entgegenhaltungen:
- EP-A- 0 186 471
- EP-A- 0 472 475
- WO-A-92/08424
- FR-A- 2 663 536
- US-A- 4 822 366

## Beschreibung

Die Erfindung bezieht sich auf eine Kniegelenkendoprothese zum Ersatz der Schienbein-Gelenkflächen, die einen am Knochen zu verankernden Lagerteil mit oberseitiger Lagerfläche und ein darauf zu befestigendes Plateau umfaßt, das die zu ersetzenden Gelenkflächen bildet. Es ist bekannt, dieses Plateau auf der Lagerfläche des Lagerteils um die Hochachse des Gelenks drehbar zu machen, damit es den bei der Beugebewegung des Knies auftretenden Rotationsbewegungen nachgeben kann. Bei einer bekannten Prothese (US-A 42 19 893) wird die zentrische Lage des Plateaus gegenüber dem Lagerteil mittels eines an dem Plateau angeordneten, nach unten ragenden, zentralen Zapfens gesichert, der in eine entsprechende Bohrung des Lagerteils eindringt. Dies hat den Nachteil, daß die Seitenführungskräfte an dem Plateau in einer Ebene angreifen, die gegenüber der Gelenkflächenebene stark versetzt ist. Auch muß der am Plateau vorgesehene Führungszapfen mit Rücksicht auf die begrenzte Festigkeit des in der Regel aus Kunststoff bestehenden Plateaus mit beträchtlicher Stärke ausgeführt sein, was dazu zwingt, am Lagerteil zur Bildung der Zapfenbohrung einen entsprechend groß dimensionierten Hohlzapfen vorzusehen, der zudem noch koaxial zu dem Drehzentrum liegen muß, was gleichfalls unerwünscht sein kann. Ein weiterer Nachteil der bekannten Konstruktion liegt darin, daß mit Rücksicht auf die Luxationsgefahr der Plateauzapfen ziemlich lang ausgebildet sein muß, was einen beträchtlichen Abstand des Plateaus über der Lagerfläche des Lagerteils beim Einsetzen verlangt, der aber nicht zur Verfügung steht, wenn der Bandapparat des Knies weitgehend intakt bleiben soll. Schließlich läßt sich bei einer solchen Ausführung nur schwer eine Luxationssicherung vorsehen.

Nach einer weiteren bekannten Prothesenausführung (EP-A 186 471) ist ein vom tibialen Lagerteil anfragender Zapfen vorgesehen, der in eine Bohrung des Plateaus eingreift. Dies hat bezüglich des bei der Operation verlangten Abstands des Plateaus über der Lagerfläche denselben Nachteil wie die zuvor beschriebene Prothese. In noch höherem Maße gilt dies für solche Prothesen, bei denen nach dem Einsetzen des Plateaus dies mit dem Lagerteil durch einen Bolzen verbunden wird (EP-A 472 475, FR-A 2 663 536). In einem Fall (US-A 4 822 366) wird dazu ein Schraubbolzen verwendet, der durch eine Bohrung des Plateaus geht, oberhalb des Plateaus einen verdickten Kopf aufweist und mit einem an seinem unteren Ende vorgesehenen Gewinde an dem tibialen Lagerteil befestigt ist, wobei ein Anschlag seine Einschraubposition festlegt. Irgendwelche Hinweise, wie die Prothese bei intaktem Bandapparat ohne wesentlichen Längsabstand der Kniegelenkteile implantiert werden kann, lassen sich diesen bekannten Prothesen nicht entnehmen. Dies gilt auch für eine weitere bekannte Prothese (WO 92/08424), bei der das Tibiaplateau durch einen von oben in den tibialen Lagerteil einzuschraubenden Bolzen gehalten wird. Dieser wirkt nicht unmittelbar mit dem Plateau zusammen, sondern über einen Gleitstein, der einerseits gegenüber dem Bolzen drehbar ist und andererseits in einem Langloch des Tibiaplateaus gleitbar ist, so daß dies sich bei der Beugung vor- und zurückbewegen kann.

Der Erfindung liegt - ausgehend von der WO-A-9208424 als nächsliegendem Stand der Technik gemäß dem Oberbegriff des Anspruchs 1 - die Aufgabe zugrunde, eine Kniegelenkendoprothese zu schaffen, die bei intaktem Bandapparat leicht implantiert werden kann.

Die erfindungsgemäße Lösung besteht in den Merkmalen des Anspruchs 1 sowie vorzugsweise denjenigen der Unteransprüche.

Die erfindungsgemäße Konstruktion hat den Vorteil großer Einfachheit. Die Seitenführungskräfte wirken auf das Plateau etwa in derselben Ebene, in der auch die Gelenkflächen liegen. Deshalb kann der Zapfen sehr niedrig gehalten sein, so daß das Plateau leicht bei erhaltenem Bandapparat zwischen den Lagerteil und die Femurkondülen eingeschoben werden kann, bis er sich um den Zapfen legt. Da die Zapfenanordnung niedrig ist, findet sie leicht Platz in dem sich sattelförmig erhebenden Zwischenkondülenbereich des Plateaus, wobei sie im Durchmesser nicht beengt ist. Die Befestigung des Kragens an dem Zapfen wird dadurch bewirkt, daß der Kragen an einem Befestigungsteil angeordnet ist, der mit einem Vorsprung des Lagerteils in der Weise zusammensteckbar ist, daß einer dieser beiden Teile als Außenteil eine Bohrung enthält, in die der andere Teil als Innenteil einsteckbar ist. Es ist ein zwischen diesen beiden Teilen wirkendes Fixiermittel vorgesehen, das aus einem Bolzen gebildet ist, der in dem Innenteil gehalten und in seiner Fixierstellung daraus radial vorspringt, um in eine Ausnehmung des Außenteils zu ragen. Diese Ausführung hat den Vorteil, daß der Bolzen in seiner Lage durch den Außenteil gesichert ist und sich also nicht im Laufe der Zeit herausarbeiten kann. Dies gilt insbesondere dann, wenn der Bolzen eine Schraube ist. In diesem Fall sollte der Außenteil eine Bohrung zur Aufnahme eines Schraubenansatzes enthalten, deren Durchmesser geringer ist als der eines an den Ansatz angrenzenden Teils der Schraube, damit diese nicht gänzlich nach außen durchtreten kann.

Vorzugsweise ist der Außenteil an dem Kragen angeordnet; jedoch kann die Anordnung auch umgekehrt gewählt sein.

Wenn der Außenteil an dem Kragen angeordnet ist, kann nach der Erfindung die Länge der Schraube größer sein als der Durchmesser des Innenteils und soll der Außenteil eine achsparallele Nut zur Aufnahme des über den Umfang des Innenteils hinausragenden Teils der Schraube aufweisen. Das Schraubenende und die Nut wirken dann winkelpositionierend zusammen, so daß Sicherheit dafür gegeben ist, daß das am Außenteil vorgesehene Werkzeugloch sich genau an derjenigen Stelle befindet, an der der Schraubenkopf zu erwarten ist.

Die Erfindung wird im folgenden näher unter Bezugnahme auf die Zeichnung erläutert, die ein vorteilhaftes Ausführungsbeispiel veranschaulicht. Es zeigen:

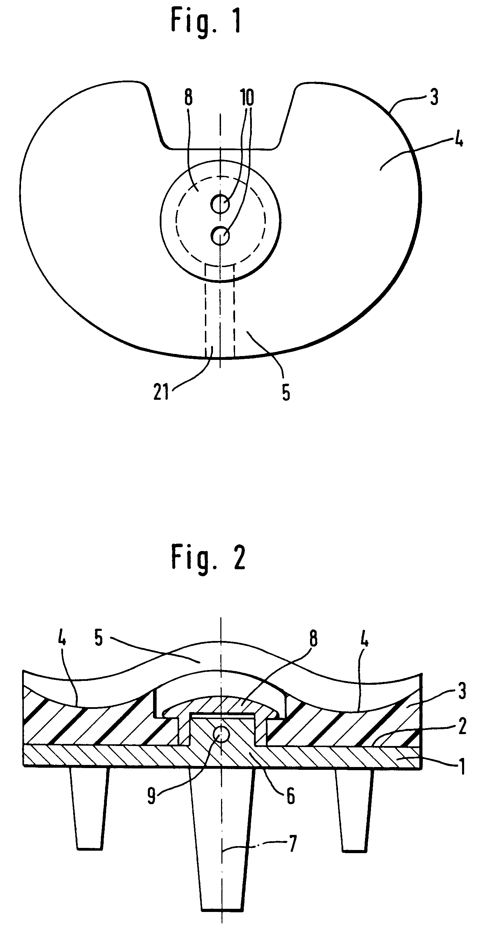

- Fig. 1: eine Draufsicht,
- Fig. 2: einen vertikalen Querschnitt und
- Fig. 3 und 4: einen Horizontalschnitt und einen Vertikalschnitt durch die Zapfenanordnung in vergrößertem Maßstab.

Das Implantat besteht im wesentlichen aus dem Lagerteil 1 mit der oberen, ebenen Lagerfläche 2 und dem Plateau 3, dessen ebene Unterfläche auf der Lagerfläche 2 aufliegt. Der Lagerteil 1 besteht aus Metall, das Plateau 3 aus Polyäthylen.

Das Plateau bildet Gelenkflächen 4, zwischen denen sich ein sattelförmiger Bereich 5 erhebt. In diesem sattelförmigen Bereich ist die Zapfenanordnung untergebracht. Diese besteht aus dem einstückig mit dem Lagerteil 1 verbundenen Innenteil 6, der zylindrisch um die Hochachse 7 ist, und einer darauf aufzusetzenden Kappe 8, die mittels einer Schraube 9 zu sichern ist. Sie enthält in der Kuppe zwei Löcher 10 für den Angriff der Finger eines Einsetzwerkzeugs.

Die Kappe 8 hat eine zylindrische Umfangsfläche 11, die passend von einer Bohrung 12 in einem Bund 13 des Plateaus 3 umfaßt wird. Die zylindrische Fläche 11 endet oben unter einem Kragen 14, dessen Unterfläche sich über den Bund 13 setzt. Sie bildet mit der Bohrung 12 die Rotationsführung für das Plateau 3. Der Kragen 14 und der Bund 13 bilden gemeinsam die Luxationssicherung für das Plateau.

Die zylindrische Fläche 11 wird von einem hohlzylindrischen Abschnitt der Kappe 8 gebildet. Dieser sitzt passend auf dem Vorsprung 6 des Lagerteils 1. Diese Teile bilden diejenigen Elemente, die weiter oben und in den Ansprüchen als Innenteil und Außenteil bezeichnet wurden.

Der Innenteil 6 enthält eine diametrale Gewindebohrung 16 zur Aufnahme der Schraube 9. An entsprechender Stelle weist der Außenteil 15 eine Bohrung 17 zur Aufnahme des Kopfs 18 der Schraube 9 auf. Wenn sich die Schraube 9 in der in Fig. 4 gezeichneten Endstellung befindet, in der der Kopf 18 in die Bohrung 17 eindringt, ist die Anordnung in ihrer Funktionsstellung gesichert. Damit die Schraube bei der Montage in diese Stellung gebracht bzw. bei der Demontage aus dieser Stellung entfernt werden kann, weist das Plateau 3 eine mit der Bohrung 17 fluchtende Bohrung 21 auf, durch die ein Schraubendrehwerkzeug mit beispielsweise einem Schraubenschlitz im Kopf 18 der Schraube 9 in Eingriff gebracht werden kann.

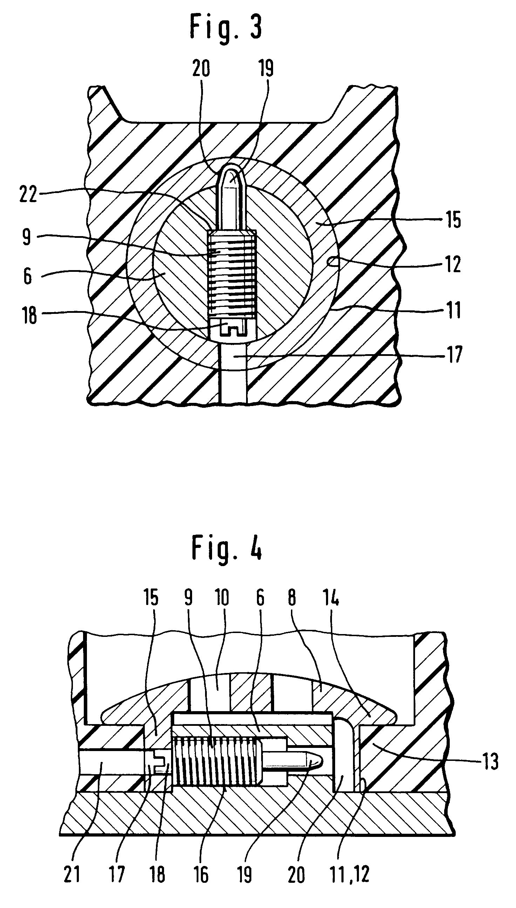

Die andere Endstellung der Schraube, in der die Kappe 8 montiert und demontiert werden kann, ist in Fig. 3 dargestellt. In dieser Stellung ragt die Spitze 19 der Schraube 9 über den Umfang des Innenteils 6 hinaus. Sie wirkt dort zusammen mit einer Axialnut 20 im Außenteil 15. Die Länge der Schraube 9 ist größer als der Durchmesser des Innenteils 6. Daraus folgt, daß stets entweder der Kopf 18 oder die Spitze 19 der Schraube über den Umfang des Innenteils 6 hinausragt.

Das Zusammenwirken der Spitze 19 mit der Nut 20 erleichtert die Montage, weil dadurch sichergestellt ist, daß die Bohrung 17 des Außenteils 15 mit der Schraube 9 fluchtet und daher beim Zurückdrehen der Schraube der Kopf 18 mit Sicherheit in die Bohrung 17 eintreten kann. Wird die Schraube soweit zurückgedreht, bis der an den Schraubenkopf 18 angrenzende, verdickte Teil, der im dargestellten Fall von dem Gewinde gebildet ist, am Rand der Bohrung 17 anschlägt, spürt der Arzt einen entsprechenden Widerstand und kann nun sicher sein, daß die gewünschte Fixierstellung erreicht wurde.

Andererseits kann Teil 8 auf den Innenteil 6 nicht aufgesetzt werden, ohne daß der Schraubenkopf 18 innerhalb des Innenteils 6 liegt; dann ist aufgrund der Länge der Schraube Gewähr dafür vorhanden, daß ihre Spitze 19 aus dem Innenteil herausschaut und führend mit der Nut 20 zusammenwirken kann. Überdies kann vorgesehen sein, daß die Schraube 9 und die sie aufnehmende Bohrung 16 mit einem zusammenwirkenden Anschlag 22 versehen sind, der dafür sorgt, daß die Spitze 19 nicht weiter als gezeigt aus dem Innenteil 6 hervorstehen kann, da anderenfalls die Aufmerksamkeit des operierenden Arztes von der Frage in Anspruch genommen wird, ob die Schraubenposition richtig zur Montage vorbereitet ist.

Nach dem Einsetzen des Lagerteils 1 kann somit das Plateau 3 über den Lagerteil geschoben werden. Danach wird die Kappe 8 eingesetzt, wobei die Nase 19 und die Nut 20 für die richtige Position sorgen. Anschließend wird ein Schraubendreher durch die Bohrungen 21 und 17 eingeführt und die Schraube bis zum Anschlag in die Sicherungsstellung gezogen.

## Patentansprüche

1. Kniegelenkendoprothese zum Ersatz der Schienbein-Gelenkflächen, die einen am Knochen zu verankernden Lagerteil (1) mit oberseitiger Lagerfläche (2) und ein darauf zu befestigendes Plateau (3) umfaßt, das auf der Lagerfläche (2) um einen Zapfen drehbar ist, dadurch gekennzeichnet, daß der von der Lagerfläche (2) sich erhebende Zapfen (6,15) mit einer Bohrung (12) in dem Plateau (3) zusammenwirkt, die einen Bund (13) aufweist, der mit einem den Zapfen oben begrenzenden, abnehmbaren Kragen (14) zusammenwirkt, und daß der Zapfen sich aus einem Vorsprung am Lagerteil (1) und einem am Kragen (14) angeordneten Befestigungsteil zusammensetzt, die in der Weise zusammensteckbar sind, daß einer dieser beiden Teile als Außenteil (15) eine Bohrung enthält, in die der andere Teil als Innenteil (6) einsteckbar ist, wobei als zwischen diesen beiden Teilen wirkendes Fixiermittel ein in dem Innenteil (6) parallel zu der Lagerfläche (2) gehaltener und in seiner Fixierstellung daraus in eine Ausnehmung des Außenteils (15) vorspringender Bolzen (9) vorgesehen ist.

2. Kniegelenkendoprothese nach Anspruch 1, dadurch gekennzeichnet, daß der Bolzen eine Schraube (9) ist und der Außenteil (15) eine Bohrung (17) zur Aufnahme eines Schraubenansatzes (18) enthält, deren Durchmesser geringer ist als der eines an den Ansatz (18) angrenzenden Teils der Schraube.

3. Kniegelenkendoprothese nach Anspruch 2, dadurch gekennzeichnet, daß der Außenteil (15) an dem Kragen (14) angeordnet ist und eine achsparallele Nut (20) zur Aufnahme eines über den Umfang des Innenteils (6) hinausragenden Teils (19) der Schraube (9) aufweist.

4. Kniegelenkendoprothese nach Anspruch 3, dadurch gekennzeichnet, daß die Länge der Schraube (9) größer ist als der Durchmesser des Innenteils (6).

5. Kniegelenkendoprothese nach einem der Ansprüche 2 bis 4, dadurch gekennzeichnet, daß die Schraube (9) und die sie aufnehmende Bohrung (16) einen Anschlag (22) zur Festlegung der Montagestellung der Schraube aufweisen.

## Claims

1. A knee-joint endoprosthesis for the replacement of tibial joint surfaces, which comprises a bearing member (1) to be anchored to the bone and having a bearing surface (2) on its upper side, and a plateau (3) to be fastened thereon and which is rotatable on the bearing surface (2) about a pivot, characterised in that the pivot (6,15) projecting from the bearing surface (2) cooperates with a bore (12) in the plateau (3), which has a shoulder (13) which co-operates with a detachable collar (14) upwardly bounding the pivot, and in that the pivot is composed of a projection on the bearing member (1) and a fastening member, mounted on the collar (14), which members can be assembled so that one of the two members comprises as the outer member (15) a bore into which the other member can be fitted as the inner member (6), wherein as a locating means acting between these two members there is provided a pin (9) which is retained in the inner member (6) parallel to the bearing surface (2) and, which in its locating position, projects therefrom into a recess of the outer member (15).

2. A knee-joint endoprosthesis according to Claim 1, characterised in that the pin is a screw (9) and the outer member (15) comprises a bore (17) to accommodate a screw extension (18), the diameter of which is smaller than that of a part of the screw adjoining the extension (18).

3. A knee-joint endoprosthesis according to Claim 2, characterised in that the outer member (15) is disposed on the collar (14) and has an axially parallel slot (20) to accommodate a part (19) of the screw (9) projecting beyond the periphery of the inner member (6).

4. A knee-joint endoprosthesis according to Claim 3, characterised in that the length of the screw (9) is greater than the diameter of the inner member (6).

5. A knee-joint endoprosthesis according any one of Claims 2 to 4, characterised in that the screw (9) and the bore accommodating it have a stop member (22) to determine the installation position of the screw.

## Revendications

1. Endoprothèse de l'articulation du genou destinée à remplacer les surfaces articulaires du tibia, comprenant une partie d'appui (1) qui est destinée à être ancrée à l'os et présente, du côté supérieur, une surface d'appui (2), et un plateau (3) qui est destiné à être fixé sur cette partie et peut pivoter sur la surface d'appui (2) autour d'un pivot, caractérisée en ce que le pivot (6,15), qui se dresse sur la surface d'appui (2), coopère avec un trou (12) formé dans le plateau (3) et présentant un rebord (13) qui coopère avec un collet amovible (14) limitant en haut le pivot, et en ce que le pivot se compose d'une saillie sur la partie d'appui (1) et d'une partie de fixation disposée sur le collet (14) qui peuvent être assemblées par le fait que l'un de ces éléments, constituant l'élément extérieur (15), contient un trou dans lequel l'autre élément, constituant l'élément intérieur (6), peut être inséré, une cheville (9), qui est maintenue dans l'élément intérieur (6) parallélement à la surface d'appui (2) et qui, dans sa position de fixation, fait saillie sur cet élément dans un creux de l'élément extérieur (15), étant prévue en tant que moyen de fixation agissant entre ces deux éléments.

2. Endoprothèse de l'articulation du genou selon la revendication 1, caractérisée en ce que la cheville est une vis (9) et l'élément extérieur (15) contient un trou (17) pour recevoir un bout de vis (18) dont le diamètre est plus petit que celui d'une partie de la vis contiguë au bout de vis (18).

3. Endoprothèse de l'articulation du genou selon la revendication 2, caractérisée en ce que l'élément extérieur (15) est disposé sur le collet (14) et présente une gorge (20) parallèle à l'axe pour recevoir une partie (19) de la vis (9) qui dépasse sur le pourtour de l'élément intérieur (6).

4. Endoprothèse de l'articulation du genou selon la revendication 3, caractérisée en ce que la longueur de la vis (9) est supérieure au diamètre de l'élément intérieur (6).

5. Endoprothèse de l'articulation du genou selon l'une quelconque des revendications 2 à 4, caractérisée en ce que la vis (9) et le trou (16) qui la reçoit présentent une butée (22) destinée à fixer la position de montage de la vis.
